# EUROPEAN PATENT SPECIFICATION

(11) **EP 2 723 266 B1**
(45) Date of publication and mention of the grant of the patent: **09.03.2016**
(21) Application number: 11730094.7
(22) Date of filing: 21.06.2011
(51) Int. Cl.: A61B 18/24, A61B 17/11

(54) **LASER CATHETER FOR BYPASS SURGERY, AS WELL AS ASSEMBLY COMPRISING SUCH A CATHETER**
LASERKATHETER FÜR BYPASS-CHIRURGIE SOWIE ANORDNUNG MIT EINEM DERARTIGEN KATHETER
CATHÉTER LASER POUR PONTAGE CHIRURGICAL ET ENSEMBLE LE CONTENANT

(43) Date of publication of application: 30.04.2014
(73) Proprietor: AMT Medical B.V., 1422 DR Uithoorn (NL)
(72) Inventor: VAN THOOR, Alexander Cornelis Elisabeth, 5991 BM Baarlo (NL)
(74) Representative: EP&C
(86) International application number: PCT/NL2011/050444
(87) International publication number: WO 2012/177117

(56) References cited:
- EP-A1- 0 750 476
- EP-A1- 1 967 152
- WO-A1-2009/123434

## Description

### Field of the invention

The invention relates to a laser catheter for bypass surgery, wherein the laser catheter comprises: a tubular arrangement comprising a tubular bundle of optical fibres having distal ends defining an emitting surface for emitting laser radiation in the distal direction of the catheter; at the distal end of the catheter, a channel extending in axial direction of the catheter and defined by the inside of the tubular arrangement, which channel is connectable to a suction source; and a grid element arranged inside the channel at a distance proximally from the emitting surface, which grid extends in transverse direction of the catheter across the channel. The invention further relates to an assembly comprising on the one hand such a catheter and on the other hand a ring member and/or a graft vessel and/or a suction source and/or an Excimer laser source. The laser catheter according to this invention as well as the assembly according to this invention can be used with the so called ELANA® operating technique, which will be described below.

### Background of the invention

A laser catheter for by-pass surgery is known from EP 750,476. This document describes the use of a laser catheter in the ELANA ® (Excimer Laser Assisted Non-occlusive Anastomosis) operating technique. For this technique, one requires a catheter and a ring, which are jointly called Elana ® Arteriotomy System.

The catheter disclosed in EP 750,476 is used for performing an ETS-anastomosis (ETS = End To Side) between a graft vessel and a target vessel. According to the ELANA ® operating technique, the graft is fixed with an end to the side of the target vessel, while the blood flow through the target vessel, also called recipient vessel, is not interrupted, i.e. blood continues to flow through the target vessel while performing arteriotomy to create the anastomosis. For this purpose, first the graft vessel is fixed to the target vessel and subsequently, after this fixation is established, the flow connection between the target vessel and graft vessel is made by removing the part of the wall of the target vessel which lies in front of the fixed end of the graft vessel. Said part of the wall of the target vessel is, according to the ELANA ® operating technique, removed by means of on the one hand a tubular arrangement of optical fibres emitting a tubular bundle of laser beams originating from the fibres and on the other hand a suction gripper provided inside the tubular arrangement of optical fibres. The tubular bundle of laser beams bums a ring shaped cut into the wall of the target vessel, resulting in a ring-based passage connecting the lumens of the graft vessel and target vessel. The ring-based wall part of the target vessel - i.e. the part lying inside said burned circle, which part is also called the "flap" - is gripped by the suction gripper and removed together with the withdrawal of the catheter after the burning operation.

In order to allow the tubular bundle of laser beams to bum a ring shaped cut into the wall of the target vessel, the laser catheter first has to be inserted into the proximal end of the graft vessel and subsequently it has to be passed through the graft vessel up to the distal end of the graft vessel.

When cutting, the surgeon cannot see the location of the ring shaped cut. The catheter inserted through the graft vessel blocks the surgeon's view onto the location of the ring shaped cut. The surgeon thus cannot see whether or not the cut is finished and the flap is completely separated from the target vessel. In case the flap is not separated completely, the flap will and can not be removed by the gripper when withdrawing the catheter from the graft vessel. A remaining flap or remaining flap parts can cause serious problems for the patient. In case the flap is not removed by the gripper, the surgeon might have to perform further actions in order to remove the flap afterwards. Consequently, it is of importance that the chance that a flap is not removed by the gripper is reduced to a minimum or said differently it is of importance to ensure that the reliability that the flap is actually removed by the gripper when withdrawing the catheter is as large as possible. In this respect, it appears that the a good cutting action and good gripping action are of importance. In practise it appears that the flap removal rate is about 85%, i.e. in 85 cases out of 100 cases the flap is correctly gripped and removed by the gripper when withdrawing the catheter from the graft vessel.

### Summary of the invention

The general object of the present invention is to provide an improved laser catheter according to the preamble of claim 1 as well as an improved assembly comprising said laser catheter and a ring member. A more specific object of the invention is to provide a laser catheter allowing an improved flap removal rate and/or allowing an improved gripping action and/or allowing an improved cutting action.
[cl 1] The above mentioned general object is according to the invention achieved by providing a laser catheter for bypass surgery, wherein the laser catheter comprises: a tubular arrangement comprising a tubular bundle of optical fibres having distal ends defining an emitting surface for emitting laser radiation in the distal direction of the catheter; at the distal end of the catheter, a channel extending in axial direction of the catheter and defined by the inside of the tubular arrangement, which channel is connectable to a suction source; and a grid element arranged inside the channel at a distance proximally from the emitting surface, which grid extends in transverse direction of the catheter across the channel; which laser catheter is characterized in that it further comprises a narrowing arranged inside the channel and, viewed in axial direction of the catheter, between the emitting surface and the grid element, in that the inner diameter of the narrowing is smaller than the outer diameter of the grid element, and in that a section of the channel extending between the narrowing and the grid element has a diameter larger than the inner diameter of the narrowing. Applicant has found that a narrowing, attached inside the channel to the tubular arrangement at a location between the grid element and the emitting surface, increases the so called flap rate. Such a narrowing can be obtained by providing a ring or tube inside the tubular arrangement. This ring or tube forms so to say an inner flange extending from the inner wall of the tubular arrangement in radial inward direction. Viewed in axial direction of the channel, this inner flange restricts the passage surface of the channel at the level of the flange. The improvement in flap rate is surprising and applicant is not certain about its explanation. Apparently, in some way the gripping and/or cutting action is improved by the narrowing. Improving the gripping action might contribute to improving the flap rate as a firmer grip allows some tearing to separate the flap from the wall of the target vessel when the ring burnt by the laser is not fully completed. Improving the gripping action might further also contribute to improving the flap rate as a firmer grip reduces the risk that the flap releases from the gripper when removing the catheter after the burning action from the graft vessel. Fact is that experiments show that providing a said narrowing allows improving the flap rate as from about 85% (without narrowing) to a range of 90-98% (for different types of narrowing) and that improving the gripping action by just increasing the suction force does not provide similar flap rate improvements.

The section of the channel extending between the narrowing and the grid element having a diameter larger than the inner diameter of the narrowing, allows, in said section, the flap being kept free from the inner wall of the tubular arrangement.

The term 'flap rate' is a kind of success rate. 'Flap rate' is defined as the % of successful flap retrievals over a range of arteriotomies (which retrievals might be obtained with laboratory tests or practical experience when applied on human or animal). A flap is successfully retrieved when after the arteriotomy and withdrawal of the laser catheter from the graft, the detached flap can be found on or in the laser catheter. In general the flap will be found in a condition hold by the gripper.

Concerning the invention as described in this application, it is noted that the term diameter does not imply it is round, it might be oval, elliptical, square, hexagonal etcetera.. For example in relation to the diameter of the narrowing, the cross section of the passage through the narrowing might be round, but it might also be oval, elliptical, square, hexagonal etcetera. The term diameter refers to the size of the passage or another part of the catheter in a direction transverse to the channel.

According to the invention a grid element is an element spanning the entire cross section of the channel and having axial passages allowing transfer of suction force from the proximal side of the grid element to the distal side of the grid element. Examples of such a grid element are a plate with one or more axial bores or other axial passages, a number of parallel bars with slits in between, a mesh, grate grating, etcetera. The function of the grid is preventing the flap from being sucked past the grid element into the tubular arrangement as this on the one hand would hinder verification whether the flap has been removed correctly and on the other hand this might cause blockage of the suctioning system.
[cl 2] According to a further embodiment of the invention, said section of the channel extending between the narrowing and the grid element and having a diameter larger than the inner diameter of the narrowing, extends from the narrowing up to the grid element. Tests showed, that this configuration provides improved flap rate, assumeably because of improved gripping action of the gripper.
[cl 3-5] According to a further embodiment, the grid element is divided in an inner part and an outer part; wherein the outer part of the grid element is defined as the part which is, when viewed in proximal direction onto the distal end of the catheter, overlapped by the narrowing (in other words lies in the shadow of the narrowing); and wherein the inner part of the grid element is defined as the part which is, when viewed in proximal direction onto the distal end of the catheter, overlapped by the passage through the narrowing (in other words lies not in the shadow of the narrowing and is visible through the passage). Effectively, this means that the surface of the inner part of the grid element has the same dimensions as the surface of the passage through the narrowing. In this embodiment the grid apertures might be provided in the inner part and/or outer part of the grid element. Providing grid apertures in the outer part ensures that at all times suction force is transferred from the proximal side of the grid element to the space at the distal side of the grid element as not all of the grid apertures in the outer part of the grid element will be closed off by a flap suctioned towards the grid element. At least some grid apertures will at any time be free from the flap even in case the flap is not fully or fully detached from the target vessel. Providing grid apertures in the inner part allows the central part of the flap to be sucked into contact with the grid part. Whereby the flap firmly lies against the narrowing, and suction force is maintained from the inner part of the grid.
[cl 6-7] According to a further embodiment, the inner edge of the narrowing could be provided with pointed pins, like a pointed serration, which pointed pins extend in a radial inward direction. Pointed pins enhance the grip onto the flap. The suction force tends to pierce one or more of these pointed pins into the flap. This appears to improve the flap rate further. According to a further embodiment, these pointed pins, at least the pointed ends of these pins, might point in a direction towards the grid to provide barbs preventing removal of the flap from the gripper in a distal direction.
[cl 8-19] According to further embodiments of the invention, the location and or dimensions of the narrowing might be specified as:
- the inner diameter of the narrowing is at most 80%, such as at most 70% or at most 60%, of the diameter of the channel; experimental tests show that as from an inner diameter of the narrowing smaller than 80% of the diameter of the channel the improvement of the flap rate is clearly noticeable, and that as from smaller than 70-60% the flap rate improvement is significant;
   and/or
- the inner diameter of the narrowing is at least 30% , such as at least 40% or at least 45%, of the diameter of the channel; experimental tests show that as from an inner diameter of the narrowing larger than 30% of the diameter of the channel the improvement of the flap rate is clearly still noticeable, and that as from larger than 40-45% the flap rate improvement is still significant;
   and/or
- the inner diameter of the narrowing is in the range of 30% to 80%, such as in the range of 40% to 60%, of the diameter of the channel; experimental tests show that for a narrowing having an inner diameter in the range of 30% to 80% of the diameter of the channel, the flap rate improvement is good noticeable, while this improvement is significant in the range of 40% to 60%;
   and/or
- viewed in the axial direction, the distance from the narrowing to the emitting surface is in the range of 0% to 60%, such as in the range of 20% to 45%, of the distance from the grid element to the emitting surface;
- viewed in the axial direction, the axial length of the narrowing is in the range of 25% to 50%, such as in the range of 35% to 40%, of the distance from the grid element to the emitting surface; this axial length can for example amount 37.5% of the distance from the grid element to the emitting surface;
- viewed in the axial direction, the distance from the narrowing to the emitting surface is in the range of 0% to 15%, such as 4% to 12%, of the inner diameter of the emitting surface; and/or
- viewed in the axial direction, the distance from the narrowing to the emitting surface is at least 0.07 mm, such as at least 0.10 mm;
   and/or
- viewed in the axial direction, the distance from the narrowing to the emitting surface is at most 0.4 mm, such as at most 0.25 mm;
   and/or
- viewed in the axial direction, the distance from the grid element to the emitting surface is at least 0.25 mm, such as at least 0.35 mm;
   and/or
- viewed in the axial direction, the distance from the grid element to the emitting surface is at most 0.6 mm, such as at most 0.5 mm;
   and/or
- viewed in the axial direction, the distance from the grid element to the narrowing is at least 0.04 mm, such as at least 0.08 mm;
   and/or
- viewed in the axial direction, the distance from the grid element to the narrowing is at most 0.5 mm, such as at most 0.4 mm or at most 0.3 mm.
[cl. 20] According to a further embodiment, the laser catheter further comprises, at the distal end of the catheter and on the outside of the tubular arrangement, a stop element enlarging the circumference of the catheter with respect to the bundle, of optical fibres.
[cl 21-31] According to a further aspect, the invention relates to an assembly comprising, on the one hand a laser catheter according to the invention and on the other hand one or more from the group of:
- a ring member, wherein the diameter of the ring member is larger than the diameter of the distal end of the tubular arrangement;
- a graft vessel having diameter dimensions allowing passage of the laser catheter through the graft vessel; wherein one end of the graft vessel might according to a further embodiment be inserted through the ring member and folded back around the ring member over an angle in the range of 90-180 degrees;
- a suction source connectable or connected to the channel;
- an excimer laser source which can be coupled to the laser catheter for transmitting laser energy generated by the laser source into the optical fibres of the laser catheter.
[cl 25-27] The graft vessel used with the present invention might be an artificial graft vessel or a biological graft vessel. According to the invention, the graft vessel can also be made from a combination of biological and artificial material. In case of a biological vessel, it is noted that this can originate from a human or animal donor - other than the patient itself -, but it can also originate from the patient itself, like a saphenous vein from the leg or an internal mammary artery from the chest. The graft vessel can according to the invention also be a vessel having a biologically cultured cell layer grown onto a supporting tube, like a harness or gauze structure.

Further it is noted that the assembly according to the invention, also in case a graft vessel originating from the patient itself, can be prepared completely outside the patient even without being connected in any manner to the patient. The assembly according to the invention can of course be made in the operation room during the operation just beside he patient itself, but it can also be made remote from the patient in a laboratory, factory or other suitable facility during or before the operation. Thus, this preparation can take place at a location where the patient is not present, but it can also take place close to the patient in the operating room.

The graft vessel thus might be separated from a human or animal body. The term 'the graft vessel being separated from a human or animal body' and the term 'the graft vessel being separated from the body of the patient' as used in this application means that the graft vessel is a separate entity not attached to a human/animal/patient body. Of coarse these term(s) do not exclude that somebody can keep it in his hand or manipulate it. These term(s) mean that the graft is, when separated, not part of the biological system of a body of a human/animal/patient.
[cl 32-33] According to a further aspect, the invention relates to a method of manufacturing a laser catheter for bypass surgery, which laser catheter comprises: a tubular arrangement comprising a tubular bundle of optical fibres having distal ends defining an emitting surface for emitting laser radiation in the distal direction of the catheter; at the distal end of the catheter, a channel extending in axial direction of the laser catheter and defined by the inside of the tubular arrangement, which channel is connectable to a suction source; and a grid element arranged inside the channel at a distance proximally from the emitting surface, which grid element extends in transverse direction of the catheter across the channel; wherein the method comprises the step of providing, viewed in axial direction of the catheter, between the emitting surface and the grid element a narrowing inside the channel. This narrowing might be provided in accordance with one or more of claims 2-20.
[cl 34] Also disclosed is a method of attaching a graft vessel to a target vessel, comprising the steps of:
- attaching a distal end of the graft vessel to the side wall of the target vessel;
- inserting a laser catheter according to the inventi into the lumen of the graft vessel;
- burning, with said laser catheter, a ring shaped opening in the side wall of the target vessel after said attaching step;
- gripping, during and after said burning step, a flap by applying a suction force to said channel of the laser catheter, which flap is defined as the part of the wall of the target vessel lying inside the ring shaped opening which is being burnt; and
- removing the laser catheter from the graft vessel by withdrawing the laser catheter in proximal direction of the graft vessel whilst continuing the gripping step and after the burning step.

In the drawings, Figures 1-5 all show prior art, to illustrate some background of the invention, and Figures 6-10 illustrate the present invention.
Fig. 1a and Fig. 1b show schematically a prior art laser catheter which can be used for the ELANA ® operation technique; Fig. 1A is a longitudinal cross section and Fig. 1B is an end view according to arrow Ib in Fig. 1A;
Fig. 2a, Fig. 2b, Fig. 2c and Fig. 2d show preliminary measures for carrying out bypass surgery with the ELANA ® operation technique;
Fig. 3a, Fig. 3b and Fig. 3c show a schematic representation of various steps in attaching the graft to a recipient vessel;
Fig. 4a, Fig. 4b and Fig. 4c illustrate the manner of function of the catheter tip during light application;
Fig. 5a and Fig. 5b show the removal of the separated part of the vessel wall - also called flap - by means of the catheter;
Fig. 6 shows, schematically and in perspective view, a longitudinal cross section of the distal end of a laser catheter according to a first embodiment of the invention;
Fig. 7 shows, schematically and in perspective view, the distal end of a laser catheter according to a second embodiment of the invention, wherein Fig. 7A shows a longitudinal cross section and Fig. 7B a perspective view;
Fig. 8 shows, schematically, the distal end of a laser catheter according to a third embodiment of the invention, wherein Fig. 8A shows, in perspective view, a longitudinal cross section and Fig. 8B a view, according to arrow VIII-B in Fig. 8A, onto the distal end of the laser catheter according to Fig. 8A;
Fig. 9A and Fig. 9B explain, highly schematically, the gripping action of a prior art laser catheter as used for the ELANA ® technique;
Fig. 10A, Fig. 10B and Fig. 10C explain, highly schematically, the gripping action of a laser catheter according to the invention.

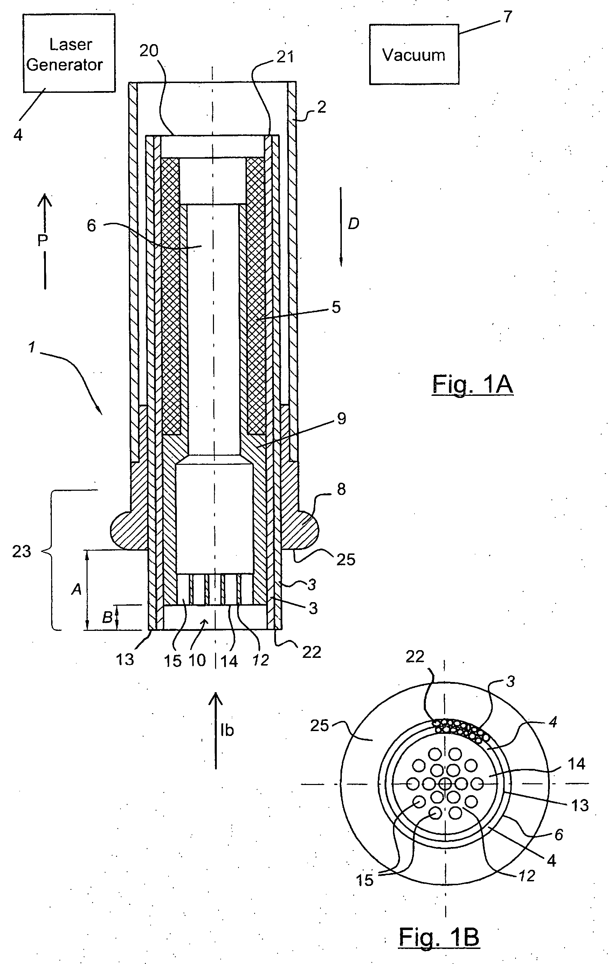

Fig. 1a and Fig. 1b show a longitudinal cross section and a bottom view of the distal region 23 of laser catheter 1. Laser catheter 1 has outer casing 2, which meets the typical demands for use in the medical field, such as easy sterilization, high flexibility and material compatibility. Outer casing 2 surrounds the tubular bundle of optical fibres 3 which are disposed in two layers, in two concentric circles in the preferred embodiment. The proximal ends 21 of the optical fibres 3 are or can be connected to laser generator 4 in a manner such that laser radiation (laser light) generated by the laser generator can be transferred into the optical fibres 3, which fibres in turn will guide the laser energy from the proximal fibre ends 21 to the distal fibre ends 22. Inner tube-shaped casing 5 surrounds bore 6, which is joined at its proximal end 20 to a low pressure source 7. At the distal region of laser catheter 1, is outer circumference-widening element 8, which has a ring-shaped cross section, with a straight stop face 25 facing in the direction of the distal end. The dimensioning of the outer-circumference widening element 8 is in general such that the outer circumference of element widening the outer circumference of laser catheter 1 has at least the diameter of the vessel through which the laser catheter is to be guided. In this way, it is ensured that the catheter tip is centered inside the vessel in a self-guiding manner by resting with its circumference on the inside area of the vessel.

In the direction of the distal end 24 of the laser catheter, the tubular bundle of optical fibres 3 project beyond the plane of the widening element 8. The optical fibres 3 for their part surround a holding means 9, which represents on the distal end a termination for the inner casing 5 but which, in particular provides, a holding device for a gripper 10 which comprises porous member 11 which leads to the low pressure prevailing inside bore 6. Bore 6 extends to the distal end of fibres 3. Referring to both Fig. 1A and Fig. 1B, the porous member is according to an advantageous arrangement a plate 14 provided with perforation holes 15, also called a grid 12. Further, Fig. 1b shows the circumference area of the element 8 widening the outer circumference of the fibres 3.

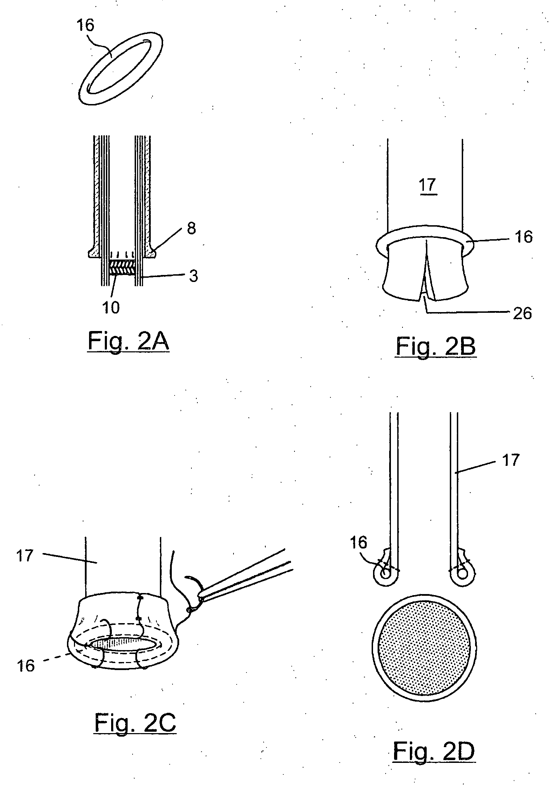

The laser catheter preferably has optical fibres densely arranged to form with their distal ends a plane circle with an outer diameter of, for example, about 2 mm. The distal ends 22 of the optical fibres 3 define a ring shaped emitting surface 13 for emitting laser radiation in the distal direction P of the catheter 1. The ring shaped emitting surface 13 is in general circular, but might also have oval, rectangular or another ring shape. In case all optical fibres emit simultaneously laser energy, a tubular bundle of laser energy/light will be emitted. In the embodiment as shown in Fig. 2B, there are 2 concentric rings 4 of optical fibres, each ring comprises a multiplicity of optical fibres arranged parallel and sideways abutting each other to obtain a dense configuration.

The porous member 11 is set inside optical fibres 3 at a distance B from the distal ends 22 of the optical fibres 3. The stop face 25 is arranged at a distance A from the distal ends 22 of the optical fibres.

For a closer description of using a laser as shown in Fig. 1, Fig. 2 to Fig. 5 illustrate schematically the manner of applying it to carry out bypass surgery, according to the Elana ® technique, without interrupting the blood flow within the blood-carrying vessel.

Fig. 2a shows basically the same laser catheter as Fig. 1, but on a more schematic level. Above this catheter a ring-shaped element 16 is shown, the diameter of which is larger than the outer diameter of the tubular bundle of optical fibres and smaller than (or the same size as) the diameter of the element 8 widening the outer circumference of the laser catheter. As known from prior art, the inner diameter of the ring (made, for example, of a body-compatible platinum-iridium alloy or pure platinum) might be about 2.6 to 2.8 mm.

In preparation for bypass surgery, the ring is slipped over a graft vessel 17 - also called graft -, which is taken from a different region of the body of the patient so that the surgical joining of such removed vessel, (such as a piece of artery) as a bypass for the blood-carrying vessel, is not complicated or prevented by the body's own rejection. The graft can also be taken from another person or an animal. Alternatively, graft vessel 17 can be an artificial vessel instead of a donor vessel taken from the patient, animal or other person.

According to Fig. 2b, subsequently the graft vessel is everted around the ring. For this purpose an incision 26 can be made into the graft vessel. The end of the graft vessel folded back around the ring against the graft vessel, is subsequently sewn in the manner shown in Fig. 2c and 2d. In this way, a stable end of the vessel is obtained which according to Fig. 2d assumes the outer contour of ring 16 in a stable form.

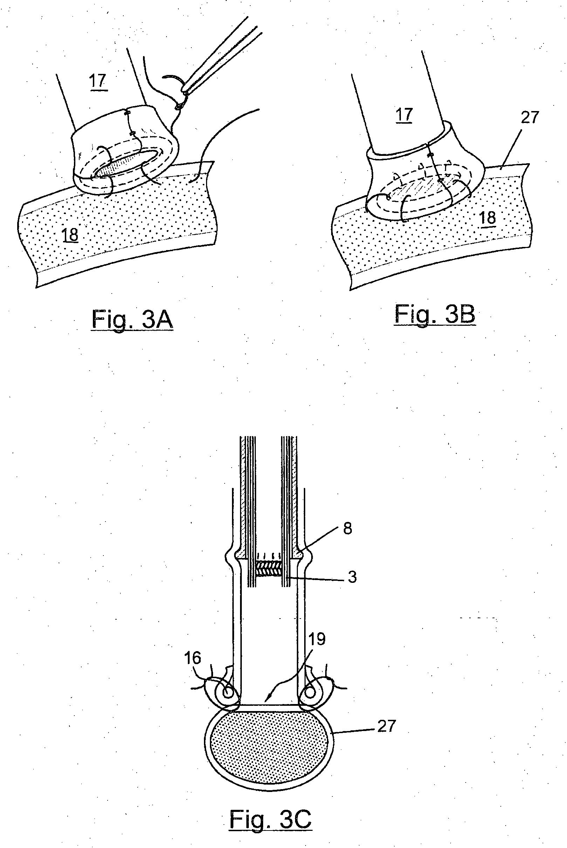

Next, the graft vessel 17, thus prepared, is joined to the outer surface of the recipient vessel 18 - also called target vessel - , by means of a ring-shaped seam, as shown in Fig. 3a. In the state according to FIG. 3b, the graft vessel 17 rests firmly on the surface of the recipient vessel 18. Then the laser catheter is guided through the inside of the graft vessel 17 in the direction of the vessel wall 27 of the target vessel 18, a portion 19 of which is to be severed. As Fig. 3c shows, due to the ring 16 the portion 19 of vessel wall 27 is, in the region lying inside the contour of ring 16, made taut for attaching the graft, in such a manner that the distal end of the laser catheter can be placed on a plane area. This has the advantage that during application of laser energy, the vessel wall part 19 is impinged evenly with laser light in the contact region with the ring shaped emitting surface 13 (formed by the distal ends 22 of the laser fibres 3).

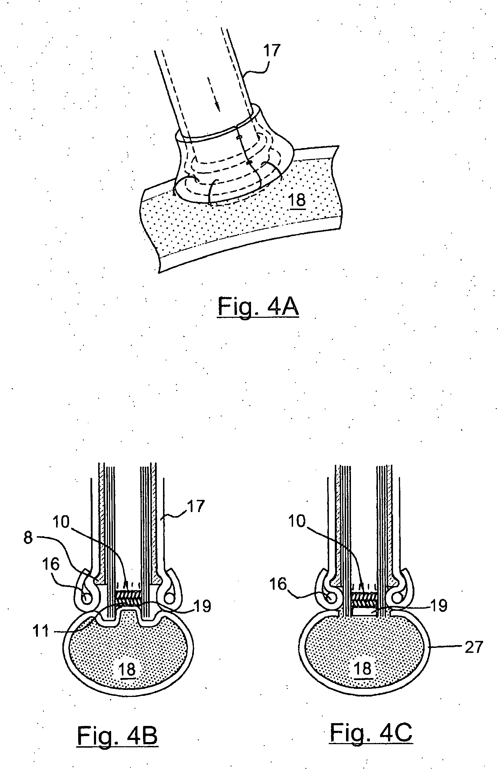

Fig. 4a shows a perspective representation of the connection of the graft vessel 17 and target vessel 18 as well as the entry of the laser catheter through the graft vessel 17. The distal end of the laser catheter is, as depicted in Fig. 4b, first guided through vessel 17 until emitting surface 13 of the laser catheter rests on the part 19 of the vessel wall 27, which part 19 is to be separated and is also called the 'flap'. Thereafter, low pressure source 7 connected to the bore 6 is activated and ensures that the flap 19 to be separated is drawn to the gripper 10 by the suction force generated by the low pressure source 7.

Now laser generator 4 is activated (in the present application, preferably an Excimer laser for generating ultra-violet radiation). The laser might be operated in pulses with a repetition frequency of e.g. 40 Hz, for about 5 seconds, so that about 200 pulses impinge upon the tissue. The distal end of the laser catheter thus slowly penetrates the lumen of the target vessel 18 until the ring 16 prevents stop edge 25 of widening element 8 - and consequently the laser catheter - from entering the lumen of target vessel any further. Thus as shown in Fig. 4c, the piece of the vessel wall 19 - the flap - is separated from the remaining wall 27 of the vessel 18, and adheres to the surface of the gripper 10.

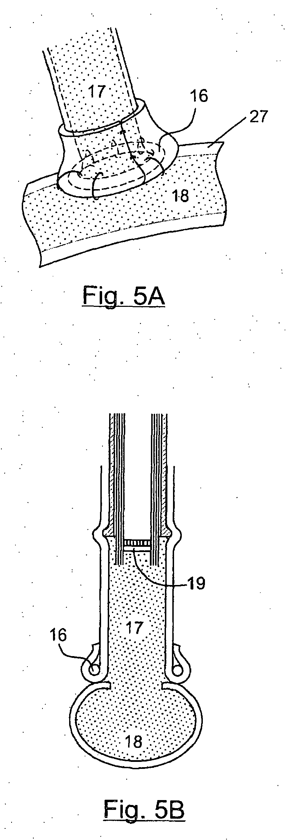

According to Fig. 5a and Fig. 5b, the blood can now flow through the graft vessel 17 after the distal end of the laser catheter has been removed from the graft vessel 17, together with the separated flap 19. Ring 16 remains at the bypass connection between the vessels 17 and 18.

By means of this ELANA technique, it is possible to conduct bypass surgery with creating a complete defined hole in blood vessels to be treated without perforating the vessel unintentionally and without interrupting the blood flow through the target vessel and without removing the pressure in the target vessel. The above described Elana ® technique as well as the invention to be described below can be applied to any intracorporal vessels, in particular, for bypass surgery on vessels in the brain and on coronary vessels of the heart.

In the above elucidation, the ring shaped element 16 is a part consisting of only a ring member. The ring shaped element 16 as shown has no protrusions or other additional members. It is however noted that also other embodiments for the ring shaped element are known or conceivable. Examples of other embodiments for the ring shaped element 16 can for example be found in WO 2009/123434 and PCT/NL2010/050778. Ring elements according to these WO 2009/123434 and PCT/NL2010/050778 have two about parallel pins which are inserted into the target vessel in order to attach the assembly of graft vessel and ring element onto the wall of the target vessel. Sewing like shown in figures 3a-3c might be superfluous with a ring element according to WO 2009/123434 or PCT/NL2010/050778. Further with a ring element according to WO 2009/123434 or PCT/NL2010/050778, in general there are made two or more incisions 26 (see fig. 2B) and the sewing as shown in Fig. 2C might not be applied. Further the flaps formed in between incision might be glued, adhered or otherwise attached flat to the wall of the recipient vessel (as shown for example in Fig. 8C of WO 2009/123434. The present invention can be applied in combination with all these variants as well as other variants not described.

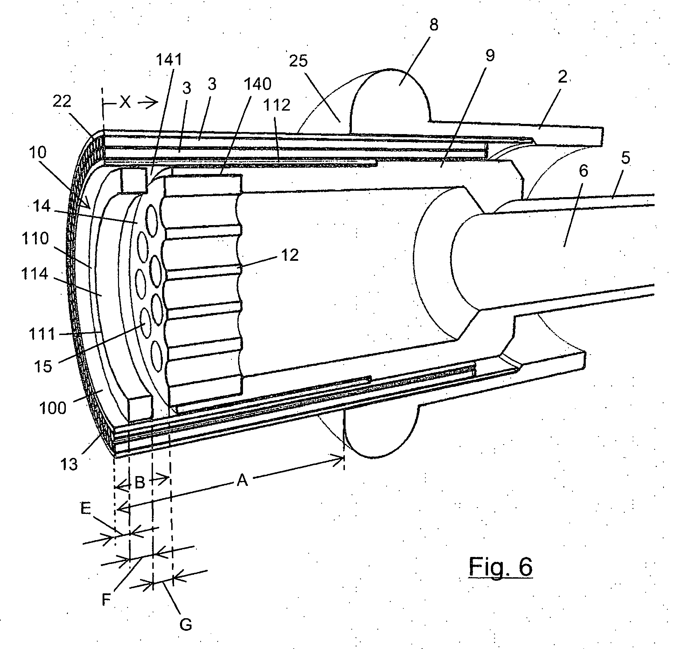

The reference numbers used in relation to the prior art laser catheter of figures 1-5 are used in figures 6-8 as well to indicate similar or same parts. As these are already elucidated, this will not be repeated. Referring to the invented laser catheters as shown in figures 6-8, the features additional in relation to the present invention will be elucidated.

Referring to the main claim, the said 'channel' 100 is defined as the channel extending from the distal ends 22 of the optical fibres 3, i.e. the emitting surface 13, in the direction of arrow X (shown Fig. 6) up to at least the grid 12. The grid 12 is thus arranged inside the channel 100. The grid 12 might define the end of the channel 100 or the channel 100 might also continue proximally of the grid 12

According to the invention the laser catheter is provided with a narrowing arranged inside the said channel 100 between the emitting surface 13 and the grid 12. The narrowing according to the invention might have different dimensions and might be provided in many different manners. Figures 6-8 show each a different variant of a narrowing 110, 120 and 130, respectively. The narrowing is in the figures 6-8 provided as in insert, which is fitted into a prior art laser catheter. It is however noted, that it is also conceivable to form the narrowing as an integral part with another part of the catheter, like the holding means 9 or the grid 12.

The narrowings 110, 120, 130 each have an inner edge 111, 121 and 131 respectively, which defines the inner diameter of the narrowing. The inner edge 131 has a serrated shape, indicating that this inner diameter can be non-circular. The grid element 12 has an outer circumference 140. As can be seen in figures 6-8, the inner edges 111, 121 and 131 of the narrowings 110, 120 and 130 all have a diameter which is smaller than the diameter of the outer circumference 140 of the grid element 12. Further, it can be seen in figures 6-8, that said channel 100 has a section 141 extending between the narrowing 110, 120, 130 and the grid element, which section has a diameter larger than the inner diameter of the narrowing.

Referring to Fig. 6, the narrowing 110 is formed by a circumferential rib inside the channel 100. This rib might be obtained by machining a tubular body to an integral member comprising the narrowing 110 and 112. However, the rib might also be obtained in another manner, for example - as shown in Fig. 6 - by inserting a ring 114 into the channel 100 and fixing the ring 114 to the channel. However, it is noted that the rib 114 can also be fixed in different manner into the channel, for example by means of a circumferential groove inside the channel into which the rib 114 engages or by means of locking ridges engaging the rib 114 in between. As can be seen in Fig. 6 the ring 114 has an outer diameter about equal to the inner diameter of the channel 100, so that a clamp fit might be obtained. In the embodiment of Fig. 6 it can further be seen that the section 141 of the channel 100, extending between the narrowing 114 and the grid 12 and having a diameter larger than the inner diameter 114 of the narrowing, might optionally extend over the full distance between the narrowing 110 and the grid 12. The rib/ring 114 forms a kind of inner flange extending from the inner wall of the channel 100 in radial inward direction.

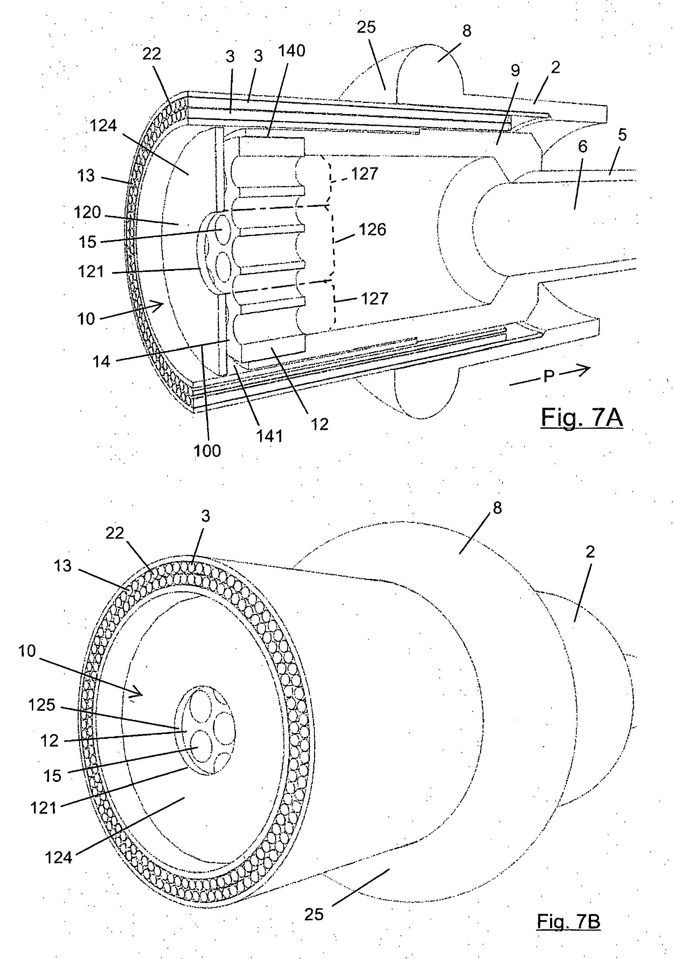

Referring to Fig. 7, the narrowing 120 is formed by a ring shaped plate arranged inside the channel 100. This ring shaped plate can be obtained in different manners, for example - as shown in Fig. 7 - by inserting a plate 124 with a central puncture 125 into the channel 100 and fixing the plate 124 to the channel. As can be seen in Fig. 7 the plate 124 has an outer diameter about equal to the inner diameter of the channel 100, so that a clamp fit might be obtained. However, it is noted that the plate 124 can also be fixed in different manner into the channel, for example by means of a circumferential groove inside the channel into which the outer edge of the plate 124 engages or by means of locking ridges engaging the outer edge of the plate 124 in between. In the embodiment of Fig. 7 it can further be seen that the section 141 of the channel 100, extending between the narrowing 120 and the grid 12 and having a diameter larger than the inner diameter 124 of the narrowing, might extend over the full distance between the narrowing 114 and the grid 12. The punctured plate 124 forms a kind of inner flange extending from the inner wall of the channel 100 in radial inward direction.

In the embodiment of Fig. 7 it can also be seen that the grid element 12 is divided in an inner part 126 and an outer part 127. The outer part 127 of the grid element 12 is defined as the part which is, when viewed in proximal direction P onto the distal end of the catheter, overlapped by the narrowing 120, in other words lies the outer part 127 lies in the shadow of the narrowing 120. The inner part 126 of the grid element 12 is defined as the part which is, when viewed in proximal direction P onto the distal end of the catheter, overlapped by the passage (puncture 125) through the narrowing 120, in other words the inner part 126 lies not in the shadow of the narrowing 120 and is visible through the passage 125 when viewing in the direction of arrow P onto the distal end of the catheter. Effectively, this means that the surface of the inner part 126 of the grid element has the same dimensions as the surface of the passage 125 through the narrowing 120. In this embodiment, grid apertures 15 are provided in the inner part 126 as well as the outer part 127 of the grid element 12. Providing grid apertures in the outer part 127 ensures that at all times suction force is transferred from the proximal side of the grid element to the space at the distal side of the grid element as a so called flap will not be able to close off all of the grid apertures in the outer part of the grid element 12. At least some grid apertures will at any time be free from the flap, even in case the flap is fully or not fully detached from the target vessel. Providing grid apertures 15 in the inner part 126 allows the central part of a so called flap to be sucked into contact with the grid 12.

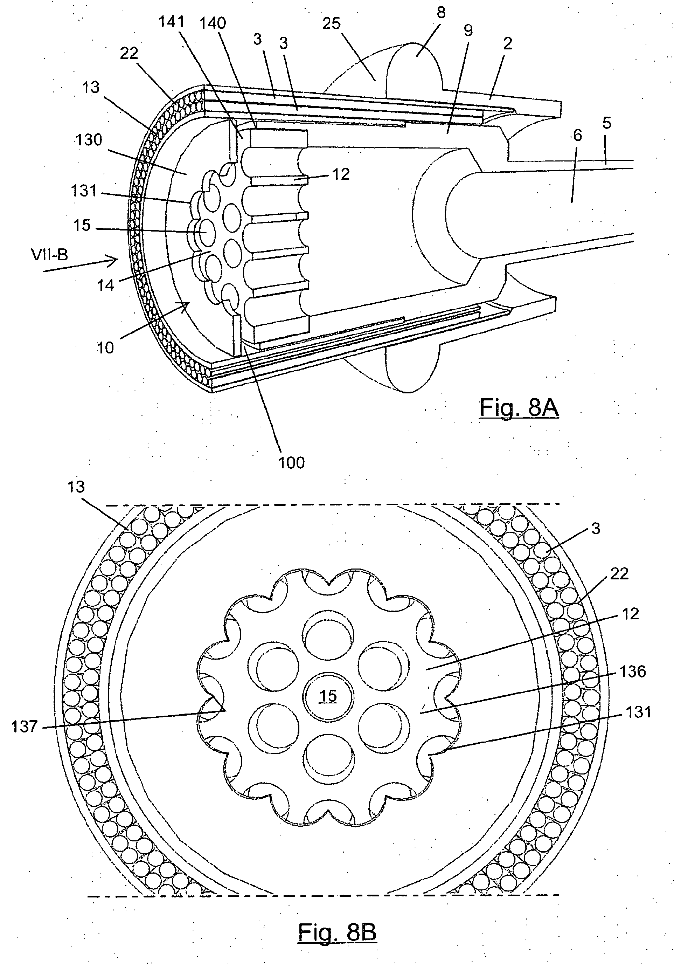

The embodiment shown in Fig. 8 is essentially the same as the embodiment shown in Fig. 7 The main difference is that the puncture 136 in the embodiment of Fig. 8 has a cross section different from circular. The inner edge 131 of the narrowing 130 is serrated to provide pointed pins 137 extending in a radial inward direction. These pointed pins provide improved gripping of the so called flap. In case the so called flap might not be fully detached from the wall of the target vessel by laser action, the remaining connection between the flap and the target vessel might by torn loose when withdrawing the catheter, which hold the flap firmly with the pins 137. This gripping action is improved further - not shown in Fig. 8 - when the pointed ends of these pins, point in a direction P towards the grid to provide barbs.

Referring to Figures 6, 7 and 8, but only shown in Fig. 6, the narrowing 110, 120, 130 is arranged, viewed in the axial direction, at a distance E from the emitting surface 13 and at a distance G from the grid 12, whilst, viewed in axial direction, the narrowing has an axial length F. As an example, the values of E, F and G are according to an embodiment of the invention: E = 0.13 mm, F = 0.15 mm and G = 0.12 mm. From this it results that B = 0.4 mm. In this embodiment the channel 100 has a circular cross section with a (inner) diameter of 1.65 - 1.7 mm, the narrowing has an inner diameter 111, 121, 131 of 0.9 mm, the outer diameter of the catheter tip (which is effectively the outer diameter of the emitting surface 13) is about 2 mm, and like in Fig. 7 grid apertures 15 were present both in the inner part 126 and the outer part 127 of the grid 12. It is however noted, that this example is not intended to limit the scope of the invention, it is only intended to provide some feeling for sizes and dimensions which a catheter according to the invention might have. The embodiment of this example has been used in comparative tests. These tests reveal in increase in flap rate from about 85% for a prior art laser catheter to over 98% for the laser catheter of this example. In these comparative tests, the only difference between the prior art laser catheter and laser catheter according to the invention was that the prior art laser catheter had no narrowing, whilst the laser catheter according to the invention had a narrowing as described in this example. To get comparative results each test involved 55 arteriotomies with the prior art laser catheter and 55 arteriotomies with the laser catheter according to the invention.

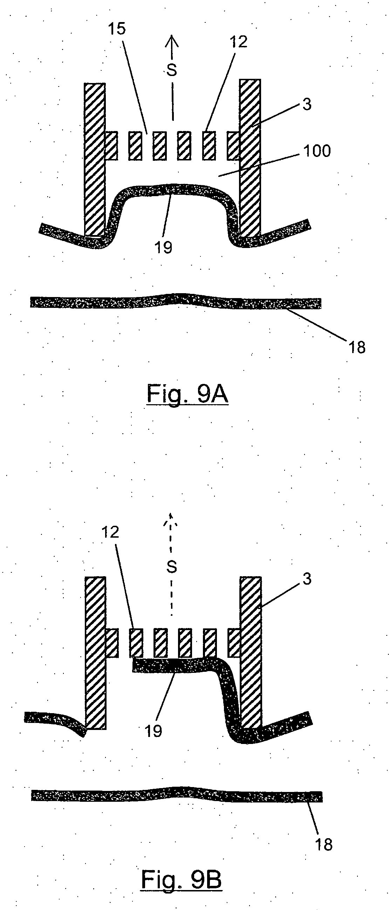

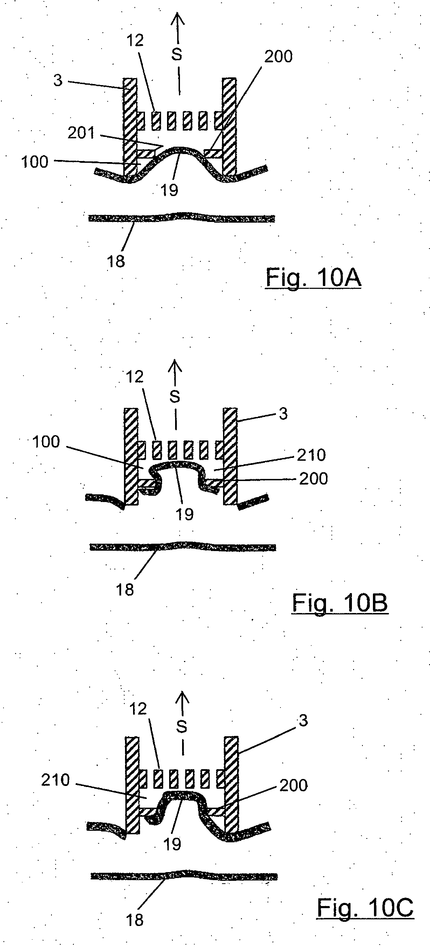

Although the inventors are not entirely sure about the explanation for the improved flap rate, it is believed that the explanation might be that with the prior art laser catheter the suction force of the gripper fails (partly) in case the flap is not fully separated from the target vessel, whilst with the laser catheter according to the invention this suction force of the gripper is maintained. This will be elucidated further with reference to Fig. 9, showing the prior art, and Fig. 10 showing the invention. These figures show highly schematically and in cross section only the grid 12, the distal ends of the optical fibres 3, a target vessel 18 and - only Fig. 10 - the narrowing 200 according to the invention. Further, arrow S indicates the suction force of the gripper. It is further noted that the distance B in these tests was about 0.4 mm and that, although the schematic drawings might suggest otherwise, the thickness of the wall of target vessel 18, and thus also the thickness of the flap 19, was about 0.3 mm, i.e. close to the size of distance B.

Fig. 9A, shows the prior art laser catheter during laser action, before any detachment of the flap 19. The suction force S creates in the channel 100 (at the distal side of the grid 12) a reduced pressure which sucks the flap 19 towards, likely against, the grid 12. When the laser action has burned the ring shaped passage through the wall of the target vessel, the flap 19 - the wall part lying inside the ring shaped passage - will be sucked against the grid so that the gripper holds it when withdrawing the catheter. Subsequently, the catheter can be inspected for the presence of the flap. If the flap is present on the grid, the arteriotomy is categorised as successful. If the flap is not present, the arteriotomy is categorised as unsuccessful. Not all prior art arteriotomies appear to be successful. The success rate appears to be about 85%.

Inventors assume that in practise, during laser action the ring shaped passage to be burned away around the flap 19, is not always completed at the same moment in time all around the flap. It is assumed that in practise it occurs that the ring shaped passage is so to say completed for example for 80%, leaving still 20% of the circumference of the flap attached to the target vessel 18. When this occurs, part of the partly separated flap 19 will be sucked against the grid 12 leaving part of the grid 12 free, see the left side of Fig. 9B. Thus the grid 12 is not fully overlapped by the flap. The consequence of this is that the suction force S becomes less effective or possibly ineffective. This has been indicated in Fig. 9B by dashing the arrow S. The grid 12 not being fully overlapped is in this situation likely promoted by the circumstance that during laser action, the flap is under elastic tension. Separating the flap partly then results in an elastic contraction, drawing the flap in the direction of the location where it is still attached to the wall of the target vessel 18, resulting in a bad contact between the distal fibre tips/emitting surface 22 and the remaining flap and so preventing an efficient ablation process.

Now turning to the laser catheter according to the invention, Fig. 10A shows the same situation as Fig. 9A. During laser action, the suction force sucks the flap 19 against the gripper. Taking into account the dimensions of the narrowing 200, the channel 100 and the thickness of the flap 19, it is believed that likely the flap will project through the narrowing 200 and rest against the grid 12.

In case of full completion of the ring shaped passage to be burned away around the flap 19 at about the same moment in time, the situation as shown in Fig. 9B is believed to occur. This situation is about the same as with the prior art, although it is believed that the inner edge 201 of the narrowing in combination with the reduced pressure prevailing at 210 improves the gripping action due to the flap being drawn against the inner edge 201 of the narrowing 200.

In case the flap 19 is not fully separated from the target vessel, the situation of Fig. 10C is believed to occur. Again for example, assume the ring shaped passage to be burned away around the flap 19 is completed for 80%, leaving 20% of the circumference of the flap attached to the graft vessel. The entire inner edge 201 of the narrowing 200 engages on the flap, so that the reduced pressure at the proximal side of the narrowing - in the part of the channel 100 between the narrowing 200 and grid 12 - is maintained, likely fully maintained. Consequently the suction force S is still effective and the gripping action is not reduced. Further, it is believed that the tension on the flap 19, at the location of the remaining 20% of the ring shaped passage to be burned away, is maintained at a level so that the burning of the laser can still be continued effectively. Further, it is believed that also in case of not continuing the laser action for the remaining 20% of the passage to be burned away, this remaining 20% of the passage can be created by just tearing it loose due to the improved gripping action. In this respect, it is noted that the gripping action could be improved further by providing pointed pins (like in Fig. 8) which can optionally be formed as barbs.

## Claims

1. Laser catheter (1) for bypass surgery,
wherein the laser catheter (1) comprises:
• a tubular arrangement comprising a tubular bundle of optical fibres (3) having distal ends (22) defining an emitting surface (13) for emitting laser radiation in the distal direction (D) of the catheter (1);
• at the distal end of the catheter (1), a channel (100) extending in axial direction of the laser catheter (1) and defined by the inside of the tubular arrangement, which channel (100) is connectable to a suction source (7);
• a grid element (12) arranged inside the channel (100) at a distance (B) proximally from the emitting surface (13), which grid element (12) extends in transverse direction of the catheter (1) across the channel (100);
**characterized,**
**in that** the laser catheter (1) further comprises a narrowing (110, 120, 130) arranged inside the channel (100) and, viewed in axial direction of the catheter (1), between the emitting surface (13) and the grid element (12);
**in that** the inner diameter of the narrowing (110, 120, 130) is smaller than the outer diameter of the grid element (12); and
**in that** a section (141) of the channel (100) extending between the narrowing (110, 120, 130) and the grid element (12) has a diameter larger than the inner diameter of the narrowing (110, 120, 130).

2. Laser catheter (1) according to claim 1, wherein said section (141) extends from the narrowing (110, 120, 130) up to the grid element (12).

3. Laser catheter (1) according to one of the preceding claims,
wherein the grid element (12) is divided in an inner part (126) and an outer part (127);
wherein the outer part (127) of the grid element (12) is defined as the part which is, when viewed in proximal direction (P) onto the distal end of the catheter (1), overlapped by the narrowing (110, 120, 130); and wherein the inner part (126) of the grid element (12) is defined as the part which is, when viewed in proximal direction (P) onto the distal end of the catheter (1), overlapped by the passage (125) through the narrowing (110, 120, 130); and
wherein the inner part (126) of the grid element (12) and/or outer part (127) of the grid element (12) are/is provided with grid apertures (15)..

4. Laser catheter (1) according to one of the preceding claims,
wherein the inner edge (131) of the narrowing (110, 120, 130) is provided with pointed pins (137), like a pointed serration, which pointed pins (137) extend in a radial inward direction;
and wherein the pointed ends of these pins (137) preferably point in a direction towards the grid element (12) to provide barbs.

5. Laser catheter (1) according to one of the preceding claims, wherein the inner diameter of the narrowing (110, 120, 130) is:
• at most 80%, such as at most 70% or at most 60%, of the diameter of the channel (100);
and/or
• at least 30% , such as at least 40% or at least 45%, of the diameter of the channel (100);
and/or
• in the range of 30% to 80%, such as in the range of 40% to 60%, of the diameter of the channel (100).

6. Laser catheter (1) according to one of the preceding claims, wherein, viewed in the axial direction:
• the distance from the narrowing (110, 120, 130) to the emitting surface (13) is in the range of 0% to 60%, such as 20% to 45%, of the distance from the grid element (12) to the emitting surface (13);
and/or
• the axial length of the narrowing (110, 120, 130) is in the range of 25% to 50%, such as in the range of 35% to 40%, of the distance from the grid element (12) to the emitting surface (13);
and/or
• the distance from the narrowing (110, 120, 130) to the emitting surface (13) is in the range of 0% to 15%, such as 4% to 12%, of the inner diameter of the emitting surface (13);
and/or
• the distance from the narrowing (110, 120, 130) to the emitting surface (13) is at least 0.07 mm, such as at least 0.1 mm;
and/or
• the distance from the narrowing (110, 120, 130) to the emitting surface (13) is at most 0.4 mm, such as at most 0.25 mm.

7. Laser catheter (1) according to claim 6, wherein, viewed in the axial direction the distance from the grid element (12) to the emitting surface (13) is:
• at least 0.25 mm, such as at least 0.35 mm;
and/or
• at most 0.6 mm, such as at most 0.5 mm.

8. Laser catheter (1) according to one of the preceding claims, wherein, viewed in the axial direction, the distance from the grid element (12) to the narrowing (110, 120, 130) is:
• at least 0.04 mm, such as at least 0.08 mm;
and/or
• at most 0.5 mm, such as at most 0.4 mm or at most 0.3 mm.

9. Laser catheter (1) according to one of the preceding claims, wherein the catheter (1) further comprises, at the distal end of the catheter (1) and on the outside of the tubular arrangement, a stop element (8) enlarging the circumference of the catheter (1) with respect to the bundle of optical fibres (3).

10. Assembly comprising:
• a laser catheter (1) according to one of the preceding claims; and
• a ring member (16);
wherein the diameter of the ring member (16) is larger than the diameter of the distal end (24) of the tubular arrangement.

11. Assembly according to claim 10, wherein the assembly further comprises a graft vessel (17) having diameter dimensions allowing passage of the laser catheter (1) through the graft vessel (17), and wherein the graft vessel (17) has diameter dimensions allowing insertion of the graft vessel (17) through the ring member (16).

12. Assembly according to claim 11, wherein one end of the graft vessel (17) is inserted through the ring member (16) and wherein the angle between the graft vessel and the part of the graft vessel folded back is in the range of 90-180 degrees.

13. Assembly according to one of claims 11-12, wherein the graft vessel (17) is:
• an artificial graft vessel;
or
• a biological vessel;
or
• separated from a human or animal body.

14. Assembly according to one of claims 10-13, wherein the assembly further comprises:
• a suction source (7) connectable or connected to the channel (100);
and/or
• an excimer laser source (4) which can be coupled to the laser catheter (1) for transmitting laser energy generated by the laser source (4) into the optical fibres (3) of the laser catheter (1).

15. Method of manufacturing a laser catheter (1) for bypass surgery, which laser catheter (1) comprises:
• a tubular arrangement comprising a tubular bundle of optical fibres (3) having distal ends (22) defining an emitting surface (13) for emitting laser radiation in the distal direction of the catheter (1);
• at the distal end of the catheter (1), a channel (100) extending in axial direction of the laser catheter (1) and defined by the inside of the tubular arrangement, which channel (100) is connectable to a suction source (7);
• a grid element (12) arranged inside the channel (100) at a distance proximally from the emitting surface (13), which grid element (12) extends in transverse direction of the catheter (1) across the channel (100);
wherein the method comprises the step of providing, viewed in axial direction of the catheter (1), between the emitting surface (13) and the grid element (12) a narrowing (110, 120, 130) inside the channel (100), which narrowing (110, 120, 130) has an inner diameter smaller than the outer diameter of the grid element (12); and wherein a section (141) of the channel (100) extending between the narrowing (110, 120, 130) and the grid element (12) has a diameter larger than the inner diameter of the narrowing (110, 120, 130); and
wherein the narrowing (110, 120, 130) is preferably provided in accordance with one or more of claims 2-9.

## Patentansprüche

1. Laserkatheter (1) zur Bypass-Operation,
wobei der Laserkatheter (1) umfasst:
• eine rohrförmige Anordnung umfassend ein rohrförmiges Bündel optischer Fasern (3), deren distale Enden (22) eine emittierende Oberfläche (13) definieren, zum Emittieren von Laserstrahlung in der distalen Richtung (D) des Katheters (1);
• an dem distalen Ende des Katheters (1) einen Kanal (100), welcher sich in axialer Richtung des Laserkatheters (1) erstreckt und durch das Innere der rohrförmigen Anordnung definiert wird, wobei der Kanal (100) mit einer Saugquelle (7) verbindbar ist;
• ein Gitterelement (12), welches innerhalb des Kanals (100) mit einem Abstand (B) proximal von der emittierenden Oberfläche (13) angeordnet ist, wobei sich das Gitterelement (12) in Querrichtung des Katheters (1) über den Kanal (100) erstreckt;
**dadurch gekennzeichnet, dass**
der Laserkatheter (1) des Weiteren eine Verengung (110, 120, 130) umfasst, die im Inneren des Kanals (100) und, betrachtet in axialer Richtung des Katheters (1), zwischen der emittierenden Oberfläche (13) und dem Gitterelement (12) angeordnet ist;
dass der Innendurchmesser der Verengung (110, 120, 130) kleiner ist als der Außendurchmesser des Gitterelements (12); und
dass ein Bereich (141) des Kanals (100), welcher sich zwischen der Verengung (110, 120, 130) und dem Gitterelement (12) erstreckt, einen größeren Durchmesser aufweist, als der Innendurchmesser der Verengung (110, 120, 130).

2. Laserkatheter (1) nach Anspruch 1, wobei sich der Bereich (141) von der Verengung (110, 120, 130) bis zu dem Gitterelement (12) erstreckt.

3. Laserkatheter (1) nach einem der vorangehenden Ansprüche, wobei das Gitterelement (12) in ein Innenteil (126) und ein Außenteil (127) unterteilt ist; wobei der Außenteil (127) des Gitterelementes (12) als der Teil definiert ist, welcher, betrachtet in proximaler Richtung (P) auf das distale Ende des Katethers (1), von der Verengung (110, 120, 130) überlappt ist; und wobei der Innenteil (126) des Gitterelements (12) als der Teil definiert ist, welcher, betrachtet in proximaler Richtung (P) auf das distale Ende des Katheters (1), von dem Durchgang (125) durch die Verengung (110, 120, 130) überlappt ist; und wobei der Innenteil (126) des Gitterelementes (12) und/oder Außenteil (127) des Gitterelementes (12) mit Gitteröffnungen (15) bereitgestellt ist/sind.

4. Laserkatheter (1) nach einem der vorangehenden Ansprüche, wobei die Innenkante (131) der Verengung (110, 120, 130) mit spitzen Stiften (137) versehen ist, wie einer spitze Verzahnung, wobei sich die spitzen Stifte (137) in einer Richtung radial nach innen erstrecken; und wobei die spitzen Enden dieser Stifte (137) vorzugsweise in einer Richtung zu dem Gitterelement (12) gerichtet sind, um Widerhaken bereitzustellen.

5. Laserkatheter (1) nach einem der vorangehenden Ansprüche, wobei der Innendurchmesser der Verengung (110, 120, 130):
• höchstens 80%, wie höchstens 70% oder höchstens 60% des Durchmessers des Kanals (100) beträgt;
und/oder
• wenigstens 30%, wie wenigstens 40% oder wenigstens 45% des Durchmessers des Kanals (100) beträgt;
und/oder
• in dem Bereich von 30 bis 80%, wie in dem Bereich von 40% bis 60% des Durchmessers des Kanals (100) liegt.

6. Laserkatheter (1) nach einem der vorangehenden Ansprüche, wobei, betrachtet in axialer Richtung:
• der Abstand der Verengung (110, 12, 130) zu der emittierenden Oberfläche (13) in dem Bereich von 0% bis 60%, wie 20% bis 45% des Abstandes des Gitterelementes (12) zu der emittierenden Oberfläche (13) liegt;
und/oder
• die axiale Länge der Verengung (110, 120, 130) in dem Bereich von 25% bis 50%, wie in dem Bereich von 35% bis 40%, des Abstandes von dem Gitterelement (12) zu der emittierenden Oberfläche (13) liegt;
und/oder
• der Abstand der Verengung (110, 120,130) in dem Bereich von 25% bis 50%, wie in dem Bereich von 35% bis 40%, des Abstandes von dem Gitterelement (12) zu der emittierenden Oberfläche (13) liegt;
und/oder
• der Abstand der Verengung (110, 120, 130) zu der emittierenden Oberfläche (13) in dem Bereich von 0 bis 15%, wie 4 bis 12%, des Innendurchmessers der emittierenden Oberfläche (13) liegt;
und/oder
• der Abstand der Verengung (110, 120, 130) zu der emittierenden Oberfläche (13) wenigstens 0,07 mm, wie wenigstens 0,1 mm beträgt;
und/oder
• der Abstand der Verengung (110, 120, 130) zu der emittierenden Oberfläche (13) höchstens 0,4 mm, wie höchstens 0,25 mm beträgt.

7. Laserkatheter (1) nach Anspruch 6, wobei, betrachtet in der axialen Richtung, der Abstandes des Gitterelementes (12) zu der emittierenden Oberfläche (13) beträgt:
• wenigstens 0,25 mm, wie wenigstens 0,35 mm;
und/oder
• höchstens 0,6 mm, wie höchstens 0,5 mm.

8. Laserkatheter (1) nach einem der vorangehenden Ansprüche, wobei, betrachtet in axialer Richtung, der Abstand des Gitterelementes (12) zu der Verengung (110, 120, 130) beträgt:
• wenigstens 0,04 mm, wie wenigstens 0,08 mm;
und/oder
• höchstens 0,5 mm, wie höchstens 0,4 mm oder höchstens 0,3 mm.

9. Laserkatheter (1) nach einem der vorangehenden Ansprüche, wobei der Katheter (1) des Weiteren an dem distalen Ende des Katheters (1) und an der Außenseite der rohrförmigen Anordnung ein Stoppelement (8) umfasst, welches den Umfang des Katheters (1) in Bezug auf das Bündel der optischen Fasern (3) vergrößert.

10. Anordnung umfassend:
• einen Laserkatheter (1) nach einem der vorangehenden Ansprüche; und
• ein Ringelement (16);
wobei der Durchmesser des Ringelementes (16) größer ist als der Durchmesser des distalen Endes (24) der rohrförmigen Anordnung.

11. Anordnung nach Anspruch 10, wobei die Anordnung des Weiteren einen Transplantationsbehälter (17) umfasst, mit Durchmesserverhältnissen, die den Durchgang des Laserkatheters (1) durch den Transplantationsbehälter (17) ermöglichen, und wobei der Transplantationsbehälter (17) Durchmesserverhältnisse aufweist, die das Einführen des Transplantationsbehälters (17) durch das Ringelement (16) ermöglichen.

12. Anordnung nach Anspruch 11, wobei ein Ende des Transplantationsbehälters (17) durch das Ringelement (16) eingeführt wird, und wobei der Winkel zwischen dem Transplantationsbehälter und dem Teil des Transplantationsbehälters, welcher zurückgefaltet ist, in dem Bereich von 90 bis 180 Grad liegt.

13. Anordnung nach einem der Ansprüche 11 bis 12, wobei der Transplantationsbehälter (17) ist:
• ein künstlicher Transplantationsbehälter;
oder
• ein biologischer Behälter;
oder
• getrennt von einem menschlichen oder tierischen Körper.

14. Anordnung nach einem der Ansprüche 10 bis 13, wobei die Anordnung des Weiteren umfasst:
• eine Saugquelle (7), welche mit dem Kanal (100) verbindbar oder verbunden ist;
und/oder
• eine Excimerlaserquelle (4), welche mit dem Laserkathether (1) verbindbar ist, um von der Laserquelle (4) erzeugte Laserenergie in die optischen Fasern (3) des Laserkatheters (1) zu übertragen.

15. Verfahren zur Herstellung eines Laserkatheters (1) für Bypassoperationen, wobei der Laserkathether (1) umfasst:
• eine rohförmige Anordnung umfassend ein rohrförmiges Bündel optischer Fasern (3), deren distale Enden (22) eine emittierende Oberfläche (13) definieren, um Laserstrahlung in der distalen Richtung des Kathethers (1) zu emittieren;
• an dem distalen Ende des Katheters (1) einen Kanal (100), welcher sich in axialer Richtung des Laserkatheters (1) erstreckt und durch das Innere der rohrförmigen Anordnung definiert wird, wobei der Kanal (100) mit einer Saugquelle (7) verbindbar ist;
• ein Gitterelement (12), angeordnet im Inneren des Kanals (100) in einem Abstand proximal von der emittierenden Oberfläche (13), wobei sich das Gitterelement (12) in Querrichtung des Katheters (1) über den Kanal (100) erstreckt;
wobei das Verfahren den Schritt des Bereitstellens einer Verengung (110, 120, 130) im Inneren des Kanals (100), betrachtet in axialer Richtung des Katheters (1), zwischen der emittierenden Oberfläche (13) und dem Gitterelement (12) umfasst, wobei die Verengung (110, 120, 130) einen Innendurchmesser aufweist, welcher kleiner ist als der Außendurchmesser des Gitterelements (12); und wobei ein Bereich (141) des Kanals (100), welcher sich zwischen der Verengung (110, 120, 130) und dem Gitterelement (12) erstreckt, einen größeren Durchmesser aufweist als der Innendurchmesser der Verengung (110, 120, 130); und
wobei die Verengung (110, 120, 130) vorzugsweise gemäß einem oder mehreren der Ansprüche 2 bis 9 bereitgestellt ist.

## Revendications

1. Cathéter laser (1) pour pontage chirurgical,
dans lequel le cathéter laser (1) comprend :
- un agencement tubulaire comprenant un faisceau tubulaire de fibres optiques (3) ayant des extrémités distales (22) définissant une surface émettrice (13) destinée à émettre un rayonnement laser dans la direction distale (D) du cathéter (1) ;
- à l'extrémité distale du cathéter (1), un canal (100) s'étendant dans la direction axiale du cathéter laser (1) et défini par la partie intérieure de l'agencement tubulaire, ledit canal (100) pouvant être raccordé à une source d'aspiration (7) ;
- un élément formant grille (12) agencé à l'intérieur du canal (100) à une distance (B) proche de la surface émettrice (13), ledit élément formant grille (12) s'étendant dans le sens transverse du cathéter (1) perpendiculairement au canal (100) ;
**caractérisé**
**en ce que** le cathéter laser (1) comprend en outre un rétrécissement (110, 120, 130) agencé à l'intérieur du canal (100) et, vu dans le sens axial du cathéter (1), situé entre la surface émettrice (13) et l'élément formant grille (12) ;
**en ce que** le diamètre intérieur du rétrécissement (110, 120, 130) est inférieur au diamètre extérieur de l'élément formant grille (12) ; et
**en ce qu'**un tronçon (141) du canal (100) s'étendant entre le rétrécissement (110, 120, 130) et l'élément formant grille (12) a un diamètre supérieur au diamètre intérieur du rétrécissement (110, 120, 130).

2. Cathéter laser (1) selon la revendication 1, dans lequel ledit tronçon (141) s'étend du rétrécissement (110, 120, 130) jusqu'à l'élément formant grille (12).

3. Cathéter laser (1) selon l'une des revendications précédentes,
dans lequel l'élément formant grille (12) se divise en une partie intérieure (126) et une partie extérieure (127) ;
dans lequel la partie extérieure (127) de l'élément formant grille (12) est définie comme étant la partie qui, vue dans le sens proximal (P) de l'extrémité distale du cathéter (1), est recouverte par le rétrécissement (110, 120, 130) ; et dans lequel la partie intérieure (126) de l'élément formant grille (12) est définie comme étant la partie qui, vue dans le sens proximal (P) de l'extrémité distale du cathéter (1), est recouverte par le passage (125) à travers le rétrécissement (110, 120, 130) ; et dans lequel la partie intérieure (126) de l'élément formant grille (12) et/ou la partie extérieure (127) de l'élément formant grille (12) est ou sont pourvues d'ouvertures de grille (15).

4. Cathéter laser (1) selon l'une des revendications précédentes,
dans lequel le bord intérieur (131) du rétrécissement (110, 120, 130) est pourvu de broches pointues (137), comme une dentelure pointue, lesdites broches pointues (137) s'étendant dans le sens radial vers l'intérieur ; et dans lequel les extrémités pointues de ces broches (137) pointent de préférence en direction de l'élément formant grille (12) pour fournir des barbelures.

5. Cathéter laser (1) selon l'une des revendications précédentes, dans lequel le diamètre intérieur du rétrécissement (110, 120, 130) égale :
- au plus 80 %, par exemple, au plus 70 % ou au plus 60 %, du diamètre du canal (100) ;
et/ou
- au moins 30 %, par exemple, au moins 40 % ou au moins 45 %, du diamètre du canal (100) ;
et/ou
- se trouve dans la plage de 30 % à 80 %, par exemple, dans la plage de 40 % à 60 %, du diamètre du canal (100).

6. Cathéter laser (1) selon l'une des revendications précédentes, dans lequel, vue dans le sens axial .
- la distance du rétrécissement (110, 120, 130) à la surface émettrice (13) se trouve dans la plage de 0 % à 60 %, par exemple, de 20 % à 45 %, de la distance de l'élément formant grille (12) à la surface émettrice (13) ;
et/ou
- la longueur axiale du rétrécissement (110, 120, 130) se trouve dans la plage de 25 % à 50 %, par exemple, dans la plage de 35 % à 40 %, de la distance de l'élément formant grille (12) à la surface émettrice (13) ;
et/ou
- la distance du rétrécissement (110, 120, 130) à la surface émettrice (13) se trouve dans la plage de 0 % à 15 %, par exemple, de 4 % à 12 %, du diamètre intérieur de la surface émettrice (13) ;
et/ou
- la distance du rétrécissement (110, 120, 130) à la surface émettrice (13) est d'au moins 0,07 mm, par exemple, d'au moins 0,1 mm ;
et/ou
- la distance du rétrécissement (110, 120, 130) à la surface émettrice (13) est d'au plus 0,4 mm, par exemple, d'au plus 0,25 mm.

7. Cathéter laser (1) selon la revendication 6, dans lequel, vue dans le sens axial, la distance de l'élément formant grille (12) à la surface émettrice (13) est :
- d'au moins 0,25 mm, par exemple, d'au moins 0,35mm ; et/ou
- d'au plus 0,6 mm, par exemple, d'au plus 0,5 mm.

8. Cathéter laser (1) selon l'une des revendications précédentes, dans lequel, vue dans le sens axial, la distance de l'élément formant grille (12) au rétrécissement (110, 120, 130) est :
- d'au moins 0,04 mm, par exemple, d'au moins 0,08 mm ; et/ou
- d'au plus 0,5 mm, par exemple, d'au plus 0,4 mm ou d'au plus 0,3 mm.

9. Cathéter laser (1) selon l'une des revendications précédentes, dans lequel le cathéter (1) comprend en outre, à l'extrémité distale du cathéter (1) et sur la partie extérieure de l'agencement tubulaire, un élément d'arrêt (8) agrandissant la circonférence du cathéter (1) par rapport au faisceau de fibres optiques (3).

10. Ensemble comprenant :
- un cathéter laser (1) selon l'une des revendications précédentes ; et
- un élément formant anneau (16) ;
dans lequel le diamètre de l'élément formant anneau (16) est supérieur au diamètre de l'extrémité distale (24) de l'agencement tubulaire.

11. Ensemble selon la revendication 10, dans lequel l'ensemble comprend en outre un vaisseau greffe (17) ayant des dimensions en diamètre permettant le passage du cathéter laser (1) à travers le vaisseau greffe (17), et dans lequel le vaisseau greffe (17) a des dimensions en diamètre permettant l'introduction du vaisseau greffe (17) à travers l'élément formant anneau (16).

12. Ensemble selon la revendication 11, dans lequel une extrémité du vaisseau greffe (17) est introduite à travers l'élément formant anneau (16) et dans lequel l' angle entre le vaisseau greffe et la partie repliée du vaisseau greffe se trouve dans la plage de 90 à 180 degrés.

13. Ensemble selon l'une des revendications 11 et 12, dans lequel le vaisseau greffe (17) est :
- un vaisseau greffe artificiel ;
ou
- un vaisseau biologique ;
ou
- séparé d'un corps humain ou animal.

14. Ensemble selon l'une des revendications 10 à 13, dans lequel l'ensemble comprend en outre :
- une source d'aspiration (7) raccordée ou pouvant être raccordée au canal (100) ;
et/ou
- une source laser à excimère (4) qui peut être couplée au cathéter laser (1) pour transmettre l'énergie laser produite par la source laser (4) dans les fibres optiques du cathéter laser (1).

15. Procédé de fabrication d'un cathéter laser (1) pour pontage chirurgical, ledit cathéter laser (1) comprenant :
- un agencement tubulaire comprenant un faisceau tubulaire de fibres optiques (3) ayant des extrémités distales (22) définissant une surface émettrice (13) destinée à émettre un rayonnement laser dans la direction distale (D) du cathéter (1) ;
- à l'extrémité distale du cathéter (1), un canal (100) s'étendant dans la direction axiale du cathéter laser (1) et défini par la partie intérieure de l'agencement tubulaire, ledit canal (100) pouvant être raccordé à une source d'aspiration (7) ;
- un élément formant grille (12) agencé à l'intérieur du canal (100) à une distance proche de la surface émettrice (13), ledit élément formant grille (12) s'étendant dans le sens transverse du cathéter (1) perpendiculairement au canal (100) ;
dans lequel le procédé comprend l'étape de fourniture, vue dans le sens axial du cathéter (1), entre la surface émettrice (13) et l'élément formant grille (12), d'un rétrécissement (110, 120, 130) à l'intérieur du canal (100), ledit rétrécissement (110, 120, 130) ayant un diamètre intérieur inférieur au diamètre extérieur de l'élément formant grille (12) ; et dans lequel un tronçon (141) du canal (100) s'étendant entre le rétrécissement (110, 120, 130) et l'élément formant grille (12) a un diamètre supérieur au diamètre intérieur du rétrécissement (110, 120, 130) ; et
dans lequel le rétrécissement (110, 120, 130) est fourni de préférence selon une ou plusieurs des revendications 2 à 9.
